# FASCICULE DE BREVET EUROPEEN

(11) **EP 3 191 160 B1**
(45) Date de publication et mention de la délivrance du brevet: **16.11.2022**
(21) Numéro de dépôt: 15763042.7
(22) Date de dépôt: 24.07.2015
(51) Int. Cl.: A61M 5/32

(54) **PROTEGE-AIGUILLE RIGIDE, PROCEDE ET MACHINE D'ASSEMBLAGE D'UN TEL PROTEGE-AIGUILLE RIGIDE**
STARRER NADELSCHUTZ, VERFAHREN UND MASCHINE ZUR MONTAGE SOLCH EINES STARREN NADELSCHUTZES
RIGID NEEDLE PROTECTOR, METHOD AND MACHINE FOR ASSEMBLING SUCH A RIGID NEEDLE PROTECTOR

(30) Priorité: 28.07.2014 FR 1457269
(43) Date de publication de la demande: 19.07.2017
(73) Titulaire: Aptar Stelmi SAS, 93420 Villepinte (FR)
(72) Inventeur: FOURNIER, Ghislain, F-17000 La Rochelle (FR); SWAL, Mickaël, F-77124 Chauconin Neufmontiers (FR)
(74) Mandataire: CAPRI
(86) Numéro de dépôt international: PCT/FR2015/052049
(87) Numéro de publication internationale: WO 2016/016556

(56) Documents cités:
- EP-A1- 1 208 861
- WO-A1-2013/104736
- FR-A1- 2 777 787
- NL-A- 8 520 389

## Description

La présente invention concerne un protège-aiguille rigide, un procédé d'assemblage d'un tel protège-aiguille rigide ainsi qu'une machine d'assemblage pour réaliser un tel procédé d'assemblage.

Les protège-aiguille rigide sont bien connus. Ils sont généralement formés d'une pièce interne en matériau élastique, tel que du caoutchouc ou un matériau thermoplastique élastomère (TPE), et dans lequel la pointe de l'aiguille est plantée pour être isolée de l'atmosphère, et d'un corps externe rigide fixé à ladite pièce interne en matériau élastique. Ce corps externe rigide renforce notamment la protection de l'utilisateur de la seringue contre une piqûre par l'aiguille en offrant une protection supplémentaire extérieure rigide qui ne peut pas ou que très difficilement être percée par l'aiguille. Le document EP1208861 décrit notamment un tel protège-aiguille rigide.

Ce type de protège-aiguille rigide est aussi utilisé dans les autoinjecteurs, auquel cas l'autoinjecteur comporte un organe de retrait du type capuchon externe, monté sur le corps de l'autoinjecteur, et qui lorsqu'il est retiré dudit corps de l'autoinjecteur, coopère avec ledit protège-aiguille rigide pour le retirer de ladite aiguille.

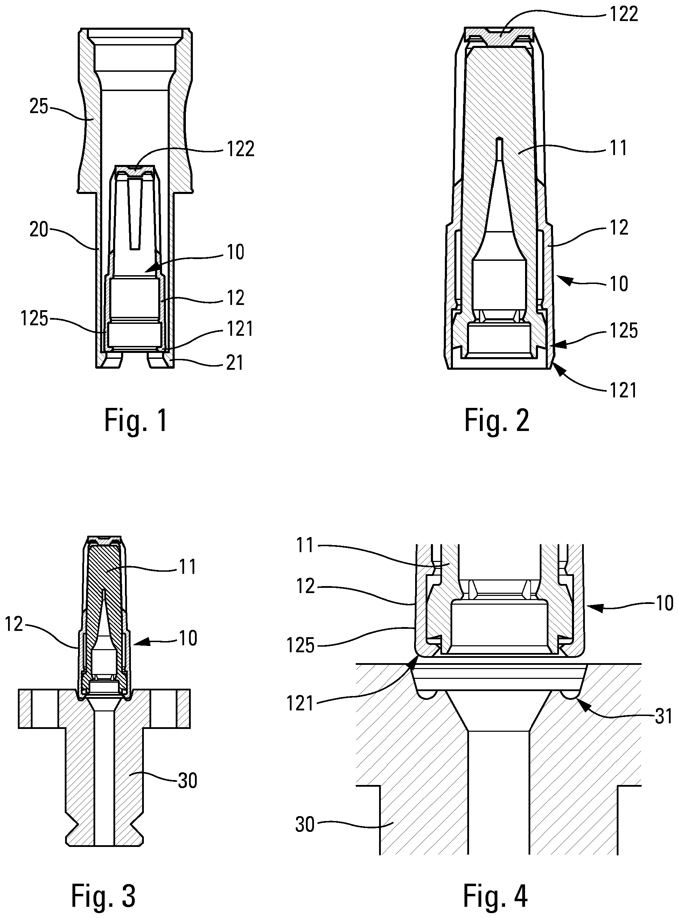

La figure 1 illustre un protège-aiguille 10 disposé dans un organe de retrait 20, selon l'état de la technique. Il est à noter que sur cette figure 1, seule le corps externe rigide 12 du protège-aiguille 10 est visible, la pièce interne en matériau élastique 11 n'étant pas représentée. Les figures 2 à 4 illustrent le procédé d'assemblage du protège-aiguille de la figure 1. La figure 2 montre la pièce interne en matériau élastique 11 insérée à l'intérieur du corps externe rigide 12. Typiquement, cette phase d'insertion se produit dans une position inversée par rapport à la position représentée sur la figure 2, avec la pièce interne en matériau élastique 11 qui tombe par gravité dans le corps externe rigide 12. Les figures 3 et 4 montrent le rabattage de l'extrémité axiale inférieure 121 du corps externe rigide 12 pour fixer ladite pièce interne en matériau élastique 11 dans ledit corps externe rigide 12. Le rabattement 121 formé pendant cette étape de rabattage s'étend radialement vers l'intérieur à partir de la surface périphérique externe 125 dudit corps externe rigide. Cette étape de rabattage se fait typiquement à chaud au moyen d'une tête de rabattage 30. On constate notamment sur la figure 4 que la forme de la cavité de rabattage 31 de la tête de rabattage 30 génère un rabattement 121 de forme externe arrondie sur le corps externe rigide 12 du protège-aiguille 10. La forme arrondie de la cavité de rabattage 31 est nécessaire pour éviter lors du rabattage d'écraser l'extrémité axiale inférieure 121 du corps externe rigide 12. Cette forme externe arrondie du rabattement 121 formé sur l'extrémité axiale inférieure du corps externe 12 peut toutefois présenter des inconvénients lorsque le protège-aiguille rigide 10 est utilisé dans un autoinjecteur. En effet, l'organe de retrait 20 dudit autoinjecteur, qui vient coopérer avec ledit protège-aiguille 10 au niveau dudit rabattement 121, peut glisser sur ledit rabattement 121 en raison de sa forme arrondie, ce peut alors empêcher le retrait dudit protège-aiguille 10 au moyen dudit organe de retrait 20.

Les documents WO 2013//104736 et NL 8 520 389 décrivent d'autres dispositifs de l'art antérieur.

La présente invention a pour but de fournir un dispositif de protection pour aiguille de seringue qui ne reproduit pas les inconvénients susmentionnés.

En particulier, la présente invention a pour but de fournir un dispositif de protection pour aiguille de seringue qui coopère de manière fiable avec un organe de retrait d'un autoinjecteur.

La présente invention a aussi pour but de fournir un procédé d'assemblage d'un dispositif de protection pour aiguille de seringue qui soit simple et peu coûteux à mettre en œuvre.

La présente invention a aussi pour but de fournir une machine d'assemblage d'un dispositif de protection pour aiguille de seringue qui permette de mettre en oeuvre ledit procédé d'assemblage.

La présente invention a donc pour objet un protège-aiguille rigide, comportant une pièce interne en matériau élastique et un corps externe rigide fixé à ladite pièce interne en matériau élastique par un rabattement de l'extrémité axiale inférieure dudit corps externe rigide, ledit rabattement s'étendant radialement vers l'intérieur à partir de la surface périphérique externe dudit corps externe rigide, la surface externe dudit rabattement formant, au niveau de son bord axialement inférieur et radialement externe, un angle vif et aigu par rapport à la surface périphérique externe dudit corps externe rigide.

Avantageusement, ladite pièce interne en matériau élastique est réalisée en caoutchouc ou en matériau thermoplastique élastomère.

La présente invention a aussi pour objet un ensemble formé d'un protège-aiguille rigide et d'un organe de retrait disposé autour dudit protège-aiguille rigide, ledit protège-aiguille rigide étant réalisé tel que décrit ci-dessus, ledit organe de retrait comportant une zone de préhension manuelle et une zone de coopération pour coopérer avec ledit protège-aiguille rigide, ladite zone de coopération comportant au moins un épaulement s'étendant radialement vers l'intérieur et coopérant avec ledit bord axialement inférieur et radialement externe dudit rabattement dudit corps externe rigide dudit protège-aiguille rigide, de sorte qu'un déplacement axial dudit organe de retrait provoque un déplacement axial dudit protège-aiguille rigide.

La présente invention a aussi pour objet une seringue comportant un réservoir et une aiguille, ladite seringue comportant un protège-aiguille rigide tel que décrit ci-dessus.

La présente invention a aussi pour objet un procédé d'assemblage d'un protège-aiguille rigide tel que décrit ci-dessus, comprenant les étapes suivantes:
- insérer ladite pièce interne en matériau élastique dans ledit corps externe rigide,
- rabattre l'extrémité axiale inférieure dudit corps externe rigide radialement vers l'intérieur au moyen d'une tête de rabattage pour former un rabattement fixant ladite pièce interne en matériau élastique dans ledit corps externe rigide,
- déformer ledit rabattement avec une tête de formage pour former sur ledit bord axialement inférieur et radialement externe dudit rabattement un angle vif et aigu par rapport à la surface périphérique externe dudit corps externe rigide.

Avantageusement, ladite étape de rabattre l'extrémité axiale inférieure dudit corps externe rigide radialement vers l'intérieur est réalisée à chaud ou par ultrasons.

Avantageusement, ladite étape de déformer ledit rabattement est réalisée à chaud ou par ultrasons.

La présente invention a aussi pour objet une machine d'assemblage d'un protège-aiguille rigide tel que décrit ci-dessus, pour mettre en œuvre le procédé tel que décrit ci-dessus, comprenant :
- une unité d'insertion pour insérer ladite pièce interne en matériau élastique dans ledit corps externe rigide,
- une tête de rabattage pour former un rabattement s'étendant radialement vers l'intérieur, pour fixer ladite pièce interne en matériau élastique dans ledit corps externe rigide,
- une tête de formage pour déformer la surface externe arrondie dudit rabattement, pour former sur ledit bord axialement inférieur et radialement externe dudit rabattement un angle vif et aigu, par rapport à la surface périphérique externe dudit corps externe rigide.

Avantageusement, ladite tête de rabattage et/ou ladite tête de formage opèrent à chaud ou par ultrasons.

Ces caractéristiques et avantages et d'autres de la présente invention apparaîtront plus clairement au cours de la description détaillée suivante, faite en référence aux dessins joints, donnés à titre d'exemples non-limitatifs, et sur lesquels :
la figure 1 est une vue schématique en section transversale d'un protège-aiguille rigide selon l'état de la technique, disposé dans un organe de retrait,
la figure 2 est une vue schématique en section transversale du protège-aiguille rigide de la figure 1, en cours d'assemblage, avec la pièce interne en matériau élastique insérée dans le corps externe rigide,
la figure 3 est une vue schématique en section transversale du protège-aiguille rigide de la figure 2, en cours d'assemblage, avec une tête de rabattage déformant l'extrémité axiale inférieure du corps externe rigide, pour fixer ladite pièce interne en matériau élastique,
la figure 4 est une vue de détail montrant la forme de la tête de rabattage et celle du rabattement réalisé par cette tête de rabattage,
la figure 5 est une vue similaire à celle de la figure 3, illustrant un mode de réalisation avantageux de la présente invention, avant actionnement d'une tête de formage,
la figure 6 est une vue similaire à celle de la figure 5, en cours d'actionnement de la tête de formage,
la figure 7 est une vue de détail de la figure 5, après actionnement de la tête de formage,
la figure 8 est une vue schématique en section transversale d'un protège-aiguille rigide selon un mode de réalisation avantageux de la présente invention, disposé dans un organe de retrait, et
les figures 9 et 10 sont des vues schématiques respectivement en perspective et en section transversale de l'ensemble de la figure 8 assemblé sur une seringue pourvue d'une aiguille.

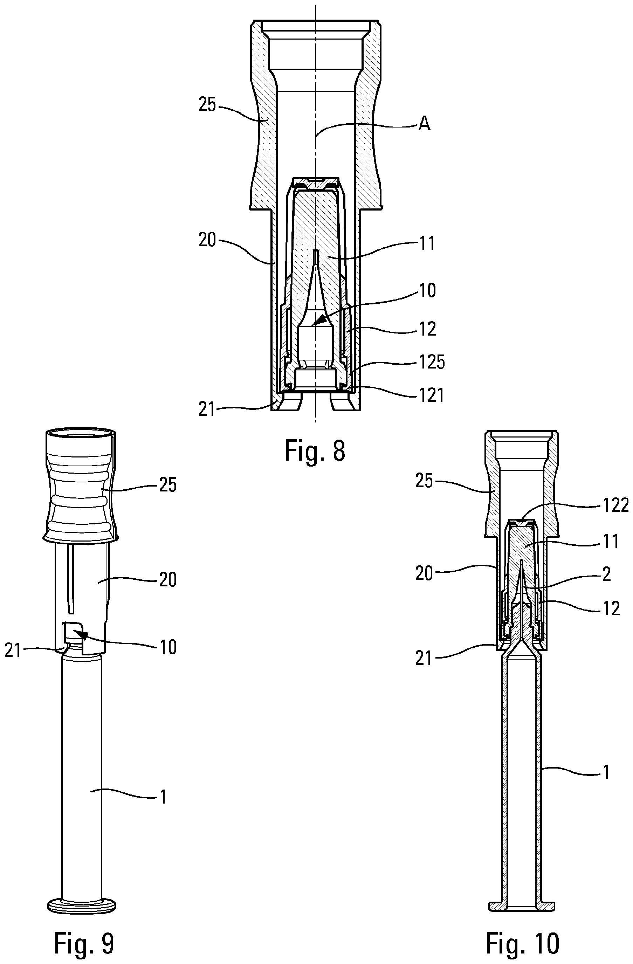

Dans la description ci-après, les termes "supérieur" et "inférieur" se réfèrent à la position droite du protège-aiguille rigide représentée sur les figures. Les termes "axial" et "radial" se réfèrent à l'axe central longitudinal A représenté sur la figure 8.

La présente invention s'applique à un protège-aiguille rigide 10, comportant une pièce interne en matériau élastique 11, tel que du caoutchouc ou un matériau thermoplastique élastomère (TPE), et un corps externe rigide 12. Ledit corps externe rigide 12 comporte une extrémité axiale inférieure et une surface d'extrémité axiale supérieure 122. Ledit corps externe rigide 12 est fixé à ladite pièce interne en matériau élastique 11 par un rabattement 121 formé sur l'extrémité axiale inférieure dudit corps externe rigide 12. Ledit rabattement 121 s'étend radialement vers l'intérieur à partir d'une surface périphérique externe 125 dudit corps externe rigide 12.

Ledit rabattement 121 est réalisé, de manière connue, par une tête de rabattage 30 comportant une cavité de rabattage 31 de forme arrondie, adaptée à déformer ladite extrémité axiale inférieure dudit corps externe rigide 12 pour former un rabattement de forme arrondie.

Selon l'invention, la surface externe dudit rabattement 121 forme, au niveau de son bord axialement inférieur et radialement externe, un angle vif et aigu, de préférence inférieur à 90°, par rapport à la surface périphérique externe 125 dudit corps externe rigide 12.

Cet angle vif et aigu est réalisé au moyen d'une tête de formage 40, qui vient modifier la forme arrondie dudit rabattement 121 telle que réalisée par ladite tête de rabattage 30.

Le rabattage au moyen de ladite tête de rabattage 30, qui donne un profil arrondi audit rabattement 121 pour assurer la fixation de ladite pièce interne en matériau élastique 11 dans ledit corps externe rigide 12, est requis, en particulier pour éviter tout risque d'écrasement dudit corps externe 12.

Ainsi, il n'est pas souhaitable de modifier la cavité de rabattage 31 de la tête de rabattage 30 pour lui donner une forme définissant un angle aigu et vif, car cela risquerait, lors du rabattage de l'extrémité axiale inférieure dudit corps externe rigide 12, de déformer ou d'écraser ledit corps externe rigide 12, ce qui pourrait altérer ledit protège-aiguille 10. Il est en effet souhaitable que lors de l'étape de rabattage, le rabattement 121 ne s'étendent que radialement vers l'intérieur, et qu'il ne se forme aucune déformation radialement vers l'extérieur sur ladite surface périphérique externe 125 dudit corps externe rigide 12. De même, il est souhaitable que lors de l'étape de rabattage, le rabattement 121 ne vienne pas comprimer ou déformer ladite pièce interne en matériau élastique 11.

L'invention prévoit donc d'utiliser la tête de rabattage 30 du procédé et de la machine d'assemblage classique, pour réaliser un rabattement de forme arrondie, et d'y ajouter une tête de formage 40, qui intervient après ladite tête de rabattage 30, pour former sur ledit rabattement arrondi ledit angle vif et aigu.

Ladite tête de formage 40 comporte donc une cavité de formage 41 définissant un angle vif et aigu, cette cavité de formage 41 venant coopérer avec ledit rabattement 121, pour former à sa surface externe un angle vif et aigu avec la surface périphérique externe 125 dudit corps externe rigide 12.

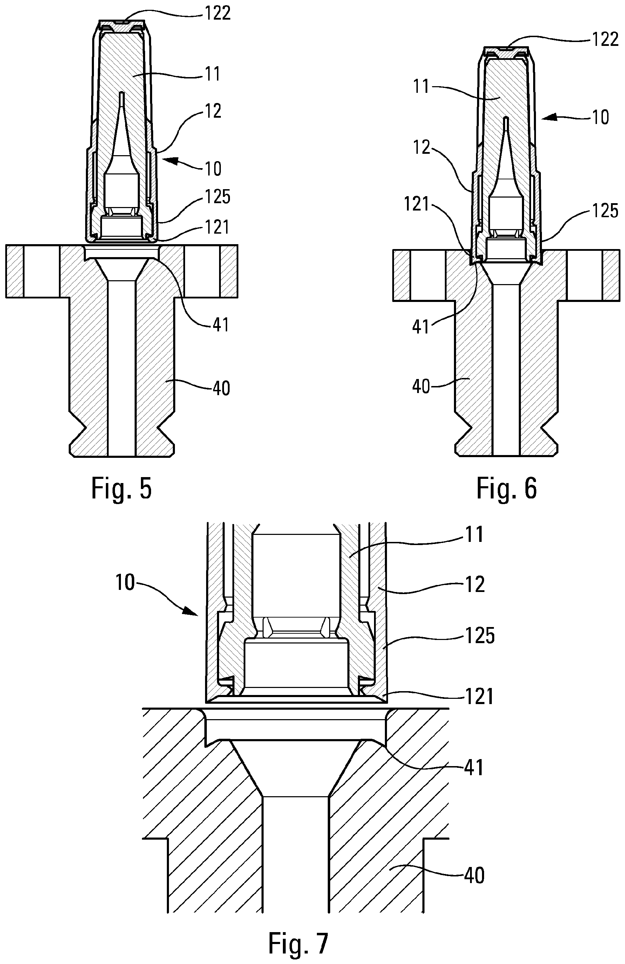

La forme de ladite cavité de formage 41 représentée sur la figure 7 est avantageuse, mais on pourrait envisager toute autre forme appropriée permettant de générer dans le rabattement 121 un angle vif et aigu au niveau de son bord axialement inférieur et radialement externe.

Avantageusement, ladite tête de rabattage 30 et ladite tête de formage 40 opèrent à chaud, ce qui permet de déformer aisément ledit rabattement 121 formé sur l'extrémité axiale inférieure dudit corps externe rigide 12. En variante, l'une ou l'autre, ou les deux têtes de rabattage 30 et de formage 40 pourraient aussi opérer par ultrasons.

Avec le rabattement 121 formé selon la présente invention avec un angle vif et aigu, il n'y a plus de risque qu'un organe de retrait 20, utilisé pour retirer le protège-aiguille rigide 10 par exemple dans un autoinjecteur, glisse sur ledit rabattement, comme c'était le cas avec le rabattement arrondi de la figure 1.

L'organe de retrait 20 comporte une zone de préhension manuelle 25, sur laquelle l'utilisateur va poser ses doigts pour exercer une traction axiale sur l'organe de retrait. Celui-ci comporte aussi une zone de coopération 21 pour coopérer avec ledit protège-aiguille rigide 10, ladite zone de coopération 21 comportant au moins un épaulement s'étendant radialement vers l'intérieur et coopérant avec ledit rabattement 121 dudit corps externe rigide 12 dudit protège-aiguille rigide 10. Ainsi, un déplacement axial dudit organe de retrait 20 provoque un déplacement axial dudit protège-aiguille rigide 10. Comme visible sur la figure 8, de par la présence de l'angle vif et aigu sur le rabattement 121 selon l'invention, ledit au moins un épaulement 21 de l'organe de retrait ne peut plus se désengager dudit rabattement 121, et le retrait du protège-aiguille rigide 10 est donc assuré en cas déplacement axial de l'organe de retrait 20.

Sur les figures 9 et 10, une seringue comportant un réservoir 1 et une aiguille 2, est pourvue d'un protège-aiguille rigide 10 selon l'invention. Avantageusement, ladite seringue est pourvue d'un ensemble formé d'un tel protège-aiguille rigide 10 et d'un organe de retrait 20. Bien entendu, la seringue comporte également un piston adapté à coulisser dans le réservoir 1, une tige de piston étant généralement prévue pour déplacer ledit piston lors de l'injection. Ces éléments ne sont toutefois pas représentés sur la figure 10, car n'intervenant pas dans la présente invention.

La présente invention a été décrite en référence à un mode de réalisation particulier, mais il est entendu qu'elle n'est pas limitée par celui-ci mais qu'au contraire l'homme du métier peut y apporter toutes modifications utiles sans sortir du cadre de la présente invention tel que défini par les revendications annexées.

## Revendications

1. Protège-aiguille rigide (10), comportant une pièce interne en matériau élastique (11) et un corps externe rigide (12) fixé à ladite pièce interne en matériau élastique (11) par un rabattement (121) de l'extrémité axiale inférieure dudit corps externe rigide (12), ledit rabattement (121) s'étendant radialement vers l'intérieur à partir de la surface périphérique externe (125) dudit corps externe rigide (12), **caractérisé en ce que** la surface externe dudit rabattement (121) forme, au niveau de son bord axialement inférieur et radialement externe, un angle vif et aigu par rapport à la surface périphérique externe (125) dudit corps externe rigide (12).

2. Protège-aiguille rigide (10) selon la revendication 1, dans lequel ladite pièce interne en matériau élastique (11) est réalisée en caoutchouc ou en matériau thermoplastique élastomère.

3. Ensemble formé d'un protège-aiguille rigide (10) et d'un organe de retrait (20) disposé autour dudit protège-aiguille rigide (10), ledit protège-aiguille rigide (10) étant réalisé selon l'une quelconque des revendications 1 ou 2, **caractérisé en ce que** ledit organe de retrait (20) comporte une zone de préhension manuelle (25) et une zone de coopération (21) pour coopérer avec ledit protège-aiguille rigide (10), ladite zone de coopération (21) comportant au moins un épaulement s'étendant radialement vers l'intérieur et coopérant avec ledit bord axialement inférieur et radialement externe dudit rabattement (121) dudit corps externe rigide (12) dudit protège-aiguille rigide (10), de sorte qu'un déplacement axial dudit organe de retrait (20) provoque un déplacement axial dudit protège-aiguille rigide (10).

4. Seringue comportant un réservoir (1) et une aiguille (2), **caractérisée en ce que** ladite seringue comporte un protège-aiguille rigide (10) selon la revendication 1 ou la revendication 2.

5. Procédé d'assemblage d'un protège-aiguille rigide (10) selon la revendication 1 ou la revendication 2, comprenant les étapes suivantes:
- insérer ladite pièce interne en matériau élastique (11) dans ledit corps externe rigide (12),
- rabattre l'extrémité axiale inférieure dudit corps externe rigide (12) radialement vers l'intérieur au moyen d'une tête de rabattage (30) pour former un rabattement (121) fixant ladite pièce interne en matériau élastique (11) dans ledit corps externe rigide (12),
- déformer ledit rabattement (121) avec une tête de formage (40) pour former sur ledit bord axialement inférieur et radialement externe dudit rabattement (121) un angle vif et aigu par rapport à la surface périphérique externe (125) dudit corps externe rigide (12).

6. Procédé selon la revendication 5, dans lequel ladite étape de rabattre l'extrémité axiale inférieure dudit corps externe rigide (12) radialement vers l'intérieur est réalisée à chaud ou par ultrasons.

7. Procédé selon la revendication 5 ou la revendication 6, dans lequel ladite étape de déformer ledit rabattement (121) est réalisée à chaud ou par ultrasons.

8. Machine d'assemblage d'un protège-aiguille rigide (10) selon la revendication 1 ou la revendication 2, pour mettre en œuvre le procédé selon l'une quelconque des revendications 5 à 7, **caractérisée en ce qu'**elle comprend :
- une unité d'insertion pour insérer ladite pièce interne en matériau élastique (11) dans ledit corps externe rigide (12),
- une tête de rabattage (30) pour former un rabattement (121) s'étendant radialement vers l'intérieur, pour fixer ladite pièce interne en matériau élastique (11) dans ledit corps externe rigide (12),
- une tête de formage (40) pour déformer la surface externe arrondie dudit rabattement (121), pour former sur ledit bord axialement inférieur et radialement externe dudit rabattement (121) un angle vif et aigu, par rapport à la surface périphérique externe (125) dudit corps externe rigide (12).

9. Machine selon la revendication 8, dans lequel ladite tête de rabattage (30) et/ou ladite tête de formage (40) opèrent à chaud ou par ultrasons.

## Patentansprüche

1. Starrer Nadelschutz (10) aufweisend ein Innenteil aus elastischem Material (11) und einen starren Außenkörper (12), der am Innenteil aus elastischem Material (11) durch eine Umlegung (121) des unteren Axialendes des starren Außenkörpers (12) befestigt ist, wobei sich die Umlegung (121) ausgehend von der äußeren Umfangsfläche (125) des starren Außenkörpers (12) radial nach innen erstreckt, **dadurch gekennzeichnet, dass** die Außenfläche der Umlegung (121) an deren axial unteren und radial äußeren Rand einen spitzen und scharfen Winkel bezogen auf die äußere Umfangsfläche (125) des starren Außenkörpers (12) bildet.

2. Starrer Nadelschutz (10) nach Anspruch 1, wobei das Innenteil aus elastischem Material (11) aus Gummi oder aus thermoplastischem Elastomermaterial hergestellt ist.

3. Anordnung, die von einem starren Nadelschutz (10) und einem Abziehelement (20), das um den starren Nadelschutz (10) herum angeordnet ist, gebildet ist, wobei der starre Nadelschutz (10) nach einem der Ansprüche 1 oder 2 hergestellt ist,
**dadurch gekennzeichnet, dass** das Abziehelement (20) einen Handgreifbereich (25) und einen Zusammenwirkungsbereich (21) aufweist, um mit dem starren Nadelschutz (10) zusammenzuwirken, wobei der Zusammenwirkungsbereich (21) wenigstens einen Ansatz aufweist, der sich radial nach innen erstreckt und mit dem axial unteren und radial äußeren Rand der Umlegung (121) des starren Außenkörpers (12) des starren Nadelschutzes (10) so zusammenwirkt, dass eine axiale Bewegung des Abziehelements (20) eine axiale Bewegung des starren Nadelschutzes (10) bewirkt.

4. Spritze aufweisend einen Behälter (1) und eine Nadel (2), **dadurch gekennzeichnet, dass** die Spritze einen starren Nadelschutz (10) nach Anspruch 1 oder 2 aufweist.

5. Verfahren zum Zusammensetzen eines starren Nadelschutzes (10) nach Anspruch 1 oder 2, umfassend die folgenden Schritte:
- Einführen des Innenteils aus elastischem Material (11) in den starren Außenkörper (12),
- Umlegen des unteren Axialendes des starren Außenkörpers (12) radial nach innen mittels eines Umlegekopfes (30), um eine Umlegung (121) zu bilden, die das Innenteil aus elastischem Material (11) im starren Außenkörper (12) befestigt,
- Verformen der Umlegung (121) mit einem Formungskopf (40), um auf dem axial unteren und radial äußeren Rand der Umlegung (121) einen spitzen und scharfen Winkel bezogen auf die äußere Umfangsfläche (125) des starren Außenkörpers (12) zu bilden.

6. Verfahren nach Anspruch 5, wobei der Schritt des Umlegens des unteren Axialendes des starren Außenkörpers (12) radial nach innen warm oder per Ultraschall erfolgt.

7. Verfahren nach Anspruch 5 oder 6, wobei der Schritt des Verformens der Umlegung (121) warm oder per Ultraschall erfolgt.

8. Maschine zum Zusammensetzen eines starren Nadelschutzes (10) nach Anspruch 1 oder 2 zur Durchführung des Verfahrens nach einem der Ansprüche 5 bis 7, **dadurch gekennzeichnet, dass** sie Folgendes umfasst:
- eine Einführeinheit zum Einführen des Innenteils aus elastischem Material (11) in den starren Außenkörper (12),
- einen Umlegekopf (30) zur Bildung einer Umlegung (121), die sich radial nach innen erstreckt, um das Innenteil aus elastischem Material (11) im starren Außenkörper (12) zu befestigen,
- einen Formungskopf (40) zur Verformung der abgerundeten Außenfläche der Umlegung (121), um auf dem axial unteren und radial äußeren Rand der Umlegung (121) einen spitzen und scharfen Winkel bezogen auf die äußere Umfangsfläche (125) des starren Außenkörpers (12) zu bilden.

9. Maschine nach Anspruch 8, wobei der Umlegekopf (30) und/oder der Formungskopf (40) warm oder per Ultraschall betrieben werden.

## Claims

1. A rigid needle guard (10) comprising an inner part (11) made of elastomeric material, and a rigid outer body (12) that is fastened to said inner part (11) made of elastomeric material via a folded-in portion (121) of the bottom axial end of said rigid outer body (12), said folded-in portion (121) extending radially inwards from the outer peripheral surface (125) of said rigid outer body (12), the rigid needle guard being **characterized in that** the outer surface of said folded-in portion (121) forms, at its axially-bottom and radially-outer edge, a sharp and acute angle relative to the outer peripheral surface (125) of said rigid outer body (12).

2. A rigid needle guard (10) according to claim 1, wherein said inner part made of elastomeric material (11) is made of rubber or made of thermoplastic elastomer material.

3. A unit formed of a rigid needle guard (10) and of a remover member (20) that is arranged around said rigid needle guard (10), said rigid needle guard (10) being made according to any one of claims 1 or 2, the unit being **characterized in that** said remover member (20) includes a grip zone (25) and a co-operation zone (21) for co-operating with said rigid needle guard (10), said co-operation zone (21) comprising at least one shoulder that extends radially inwards and that co-operates with said axially-bottom and radially-outer edge of said folded-in portion (121) of said rigid outer body (12) of said rigid needle guard (10), such that axial movement of said remover member (20) causes axial movement of said rigid needle guard (10).

4. A syringe comprising a reservoir (1) and a needle (2), **characterized in that** said syringe includes a rigid needle guard (10) according to claim 1 or claim 2.

5. An assembly method for assembling a rigid needle guard (10) according to claim 1 or claim 2. the method comprising the following steps:
- inserting said inner part (11) made of elastomeric material into said rigid outer body (12),
- folding the bottom axial end of said rigid outer body (12) radially inwards by means of a folder head (30), so as to form a folded-in portion (121) that fastens said inner part (11) made of elastomeric material in said rigid outer body (12),
- deforming said folded-in portion (121) with a shaper head (40) so as to shape, on said axially-bottom and radially-outer edge of said folded-in portion (121), a sharp and acute angle relative to the outer peripheral surface (125) of said rigid outer body (12).

6. A method according to claim 5, wherein said step of folding the bottom axial end of said rigid outer body (12) radially inwards is performed by heat or by ultrasound.

7. A method according to claim 5 or claim 6, wherein said step of deforming said folded-in portion (121) is performed by heat or by ultrasound.

8. An assembly machine for assembling a rigid needle guard (10) according to claims 1 or claim 2, for implementing the method according to any one of claims 5 to 7, the machine being **characterized in that** it comprises:
- an inserter unit for inserting said inner part (11) made of elastomeric material into said rigid outer body (12)
- a folder head (30) for forming a folded-in portion (121) that extends radially inwards, so as to fasten said inner part (11) made of elastomeric material in said rigid outer body (12),
- a shaper head (40) for deforming the rounded outer surface of said folded-in portion (121) so as to shape, on said axially-bottom and radially-outer edge of said folded-in portion (121), a sharp and acute angle, relative to the outer peripheral surface (125) of said rigid outer body (12).

9. A machine according to claim 8, wherein said folder head (30) and/or said shaper head (40) operate by heat or by ultrasound.
